# EUROPEAN PATENT SPECIFICATION

(11) **EP 2 080 480 B1**
(45) Date of publication and mention of the grant of the patent: **22.06.2011**
(21) Application number: 09150607.1
(22) Date of filing: 15.01.2009
(51) Int. Cl.: A61B 17/02, A61B 17/00

(54) **Temporary sternum retractor device**
Temporäre Sternumretraktorvorrichtung
Dispositif de rétraction temporaire du sternum

(30) Priority: 18.01.2008 IT FI20080007
(43) Date of publication of application: 22.07.2009
(73) Proprietor: Futura Engineering S.r.l., 06011 Città di Castello (PG) (IT)
(72) Inventor: Bucefari, Massimo, 52100, Arezzo (IT); Santini, Francesco, 37125, Parona (Verona) (IT)
(74) Representative: Bardini, Marco Luigi

(56) References cited:
- EP-A- 1 878 390
- US-A1- 2006 235 278

## Description

The present invention relates to the sector of devices for use in surgery; in particular, the object of the present invention is a device for temporarily holding the sternal stumps spread apart in cardiosurgical procedures.

As is known, during the final stages of cardiosurgical procedures, there may be sometimes even prolonged periods of left and/or right ventricular dysfunction as a consequence of preoperative clinical conditions (ventricular function, bronchopneumonia, renal dysfunction, etc) and/or lesions correlated with the procedure underway (myocardial protection modalities, extracorporeal circulation, intraoperative injuries, etc). These dysfunctions are often refractory to common resuscitation manoeuvres (catecholamine infusion, diuretics, intra-aortic counterpulsation, etc).

In such circumstances, it may prove unfeasible to close the sternum, especially when the cardiac dysfunction is associated with severe myocardial, pulmonary or mediastinal tissue oedema. This is because the extrinsic compression exerted by the sternum further worsens the already strained cardiopulmonary function of the engorged and congested mediastinal organs, with potentially severe haemodynamic and respiratory consequences, that are sometimes even incompatible with survival.

In the above circumstances, postponing the closure of the sternum is now considered an accredited and potentially indispensable manoeuvre, both in routine clinical experience and in the literature.

Other indications for delaying the closure of the sternum include refractory mediastinal bleeding, malignant ventricular arrhythmias correlated with myocardial oedema, and external pulmonary emphysema with potentially severe extrinsic compression even on a normally-functioning heart.

Postponing the closure of the sternum does not simply consist in leaving the sternum open. Given the above-mentioned premises, the sternal stumps (after longitudinal incision of the sternum) need to be kept spaced apart not only to reduce their extrinsic compression on the mediastinal structures as far as possible, but also to avoid the sharp profiles of the bone stumps coming into contact with the heart (right ventricle) and lung and causing traumatic lesions.

The amplitude of the artificially-induced gap between the two sternal stumps may need to be varied to suit the circumstances, but at the beginning of the procedure it is generally between 8 and 10 cm for an adult individual of average size.

In the past, endotracheal tubes, or the tubes of the extracorporeal circulatory circuit used for the patient during the surgical procedure, or plastic syringes, etc, have been used to establish and maintain this artificial divarication by inserting them between the two sternal stumps to keep the thoracic cavity open. Once the retractor device had been positioned, the surgical site was covered with a synthetic membrane stitched along its edges to prevent infections and contaminations.

These makeshift and sometimes rudimental retractor devices have often given rise to complications correlated with their stability, dimensions and the sturdiness of the materials used. For instance, a syringe with its edges cut to the required shape may become sharp and, as a consequence, potentially traumatic for the surface of the right ventricle lying underneath. In fact, traumatic lesions of the anterior surface of the right ventricle correlated with this risk have been reported.

In addition to the inadequacy of the materials, the devices used in the past to artificially divaricate the sternal stumps were simply inserted between the stumps without the opportunity to adjust the distance between them. As is self-evident to a person skilled in the art, there may be two reasons for needing to adjust this distance:
1. if the patient's haemodynamic conditions become worse, it may be necessary to further widen the gap between the sternal stumps, thereby further reducing the pressure on the mediastinal organs;
2. if the patient's haemodynamic conditions improve, it may be useful to gradually reduce the distance between the stumps, also with a view to testing the patient's tolerance of any final closing manoeuvre.

With the methods used to date, both such situations demanded the reopening of the surgical lesion (with the related reiterated risk of infection), removing the object inserted as a retractor (a syringe or other object) and replacing it with another, shorter or longer object, according to need.

Although they are considered "lifesavers", none of the retractor systems used to date have ever been validated by the competent authorities (FDA, CE, etc.).

US2006/235278 discloses an apparatus according to the preamble of claim 1 for expanding a chest cavity including an expansion assembly configured to engage the sternal stumps comprising a pair of jaws and an actuating mechanism connected to the jaws and placed therebetween for controlling their mutual closing and opening movements. EP1878390 also discloses a sternal retractor of similar type in which one of the jaws is attached to a rod movable within a handle supporting the other jaw. In both cases the actuating means, which are of any known type, take effect in the sternal stumps divarication direction.

The object of the present invention is to provide a temporary sternum retractor device for use in the case of it proving necessary to postpone the closure of the sternum after a cardiosurgical procedure, that enables the problems relating to maintaining and controlling the extent of the sternum divarication to be safely and efficiently overcome, while avoiding the risks relating to the use of makeshift devices as in the past.

A particular object of the present invention is to provide a retractor device of the above-mentioned type that can be firmly attached to the sternal stumps, which respond to the ventilator-induced respiratory movements, so that it cannot be displaced and consequently damage the mediastinal organs.

A further object of the present invention is to provide a retractor device of the above-mentioned type that can be configured to have a low profile so that it does not extend beyond the outer edges of the skin incision in order to allow for the lesion to be closed with a synthetic membrane to maintain sterile conditions at the surgical site.

These advantages are achieved by the temporary sternum retractor according to the present invention, the essential characteristics of which are stated in claim 1.

Further characteristics and advantages of the temporary sternum retractor according to the present invention will be apparent from the following description of embodiments thereof, given here as non-limiting examples with reference to the attached drawings, wherein:
figure 1 shows a perspective view of a first embodiment of the temporary sternum retractor device according to the present invention in its closed condition;
figure 2 shows the retractor device of figure 1 in its fully-opened condition;
figure 3 is a side view of the retractor device according to the invention taken along arrow F in figure 2;
figure 4 shows the retractor according to the invention in a view that is the reverse of the one in figure 2;
figure 5 is a perspective view of a second embodiment of the temporary retractor device not according to the invention in a closed condition;
figure 6 is a view of the retractor device of figure 5 in a fully-opened condition;
figure 7 is a cross-sectional view of the closed device in figure 5;
figure 8 is a cross-sectional view of the opened device in figure 6.

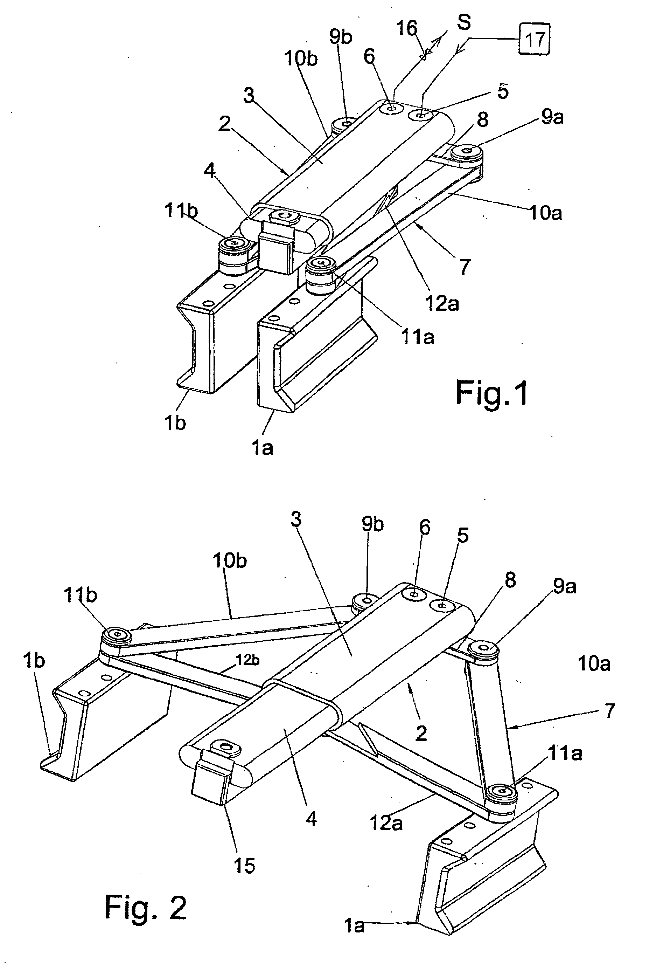

With reference to figure 1, the sternum retractor device according to a first embodiment of the invention comprises a pair of parallel and opposing jaws 1 a, 1b, with a substantially U-shaped cross-section configured in such a way to contain the two sternal stumps and afford them an abutment surface on which the force needed to keep them spread apart is applied. The two jaws 1 a, 1 b are attached to an actuator device generically indicated by the numeral 2 and comprising a fixed part in the form of a tubular sleeve 3 that is closed at one end and has a very flat oval cross-section, in which a movable part in the form of a piston 4 is slidingly engaged in a watertight manner. An actuator fluid S is inserted in or extracted from the chamber inside the sleeve 3, delimited by the piston 4, through inlet and outlet ports 5 and 6 provided on the sleeve 3.

The tubular sleeve 3 lies parallel to the two jaws 1a, 1b and the actuator device 2 is connected thereto by means of an articulated mechanism generically indicated by the numeral 7. The articulated mechanism 7 comprises a bar 8 fixed crosswise to the tubular sleeve 3 and substantially at the end of said sleeve opposite from the end in which the piston 4 slides. The bar 8 extends laterally from both sides of the sleeve 3 and its ends are pivotally connected through pins 9a, 9b to the ends of respective first arms 10a, 10b. The first arms 10a, 10b are in turn revolvingly connected at their opposite ends with the respective jaws 1 a, 1 b through pins 11 a, 11 b integral to these jaws. Therefore, the first arms 10a, 10b extend along the sides of the tubular sleeve when the retractor device according to the invention is in its closed condition, as shown in figure 1.

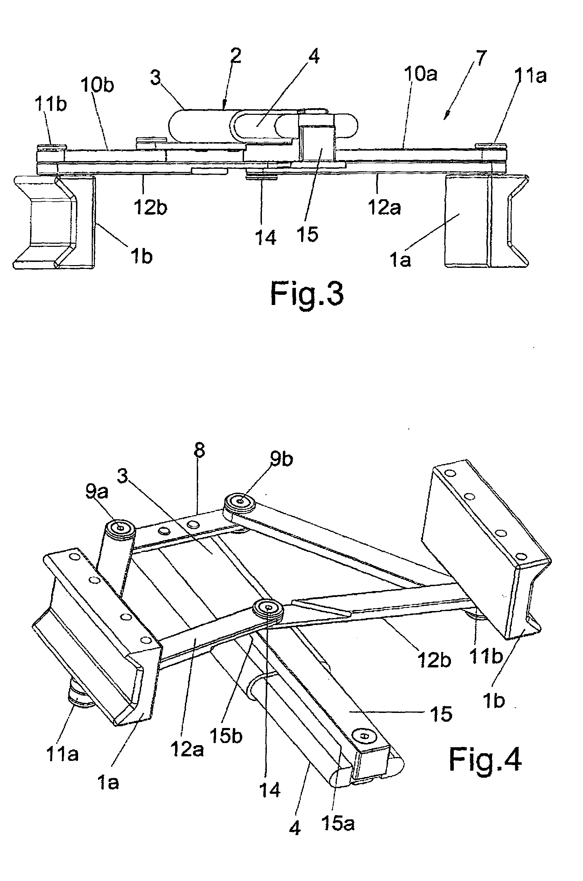

The articulated mechanism 7 also comprises second arms 12a, 12b, with ends pivotally engaging with the pins 11 a, 11 b, while their opposite ends both engage with the same pin 13, shown in figure 4, integrally attached to the piston 4. More precisely, with reference to figure 4, the pin 14 projects perpendicularly from a bracket 15 extending along the piston 4 and at a distance therefrom that is greater than the thickness of the sleeve 3, so as to enable the displacement of the sleeve 3 between the piston 4 and the bracket 15. The end of the bracket 15 is attached to the free end of the piston 4 and, on the side facing towards the piston, it is formed with two shoulders indicated as 15a and 15b, with which it abuts against the free edge of the sleeve 3 and the crosswise bar 8, respectively, when the retractor device is in its closed position.

The axes of the pins 9a,b, 11 a,b and 14 lie parallel to one another.

When the actuator fluid is fed to the sleeve 3, the piston 4 is displaced axially to emerge from the sleeve, causing the jaws 1a, 1b to move away in a direction orthogonal to the direction of said axial displacement of the piston 4 due to the effect of the articulated mechanism 7. In fact, the displacement of the piston has the effect of making the pin 14 slide axially too, extending both the second pair of arms 12a,b relative to the piston 4, and the first pair of arms 10a,b relative to the tubular sleeve 3, up to a maximally divaricated position substantially corresponding to the one shown in figures 2, 3 and 4.

Extracting the actuator fluid from the sleeve 3 causes the piston 4 to withdraw progressively in the sleeve 3 and, as a consequence, the two jaws 2a,b to get close to one another until they return to the closed position shown in figure 1.

In use, the sternum retractor device according to the present invention is attached to the two sternal stumps, which engage in the jaws 1 a,b, then actuator fluid is fed and the device is divaricated until it reaches the position best suited to the specific needs of the case. Given the kinematic configuration adopted, the opening force increases rapidly with the opening of the mechanism: in fact, a progressively greater force needs to be exerted on the two sternal stumps to open the rib cage. As the patient's conditions improve, the divarication of the device can be progressively reduced by withdrawing some of the actuator fluid from the sleeve 3.

The actuator fluid can advantageously consist of a biocompatible liquid, e.g. a physiological saline solution of known type compatible with this particular usage. The biocompatible liquid can be fed manually in the sleeve 3, by means of a syringe for instance, generally shown in figure 1 and indicated as 17, and withdrawn by opening a shut-off valve 16 located at the outlet from the sleeve 3 and exploiting the force exerted by the patient's rib cage which tend to close the opening in the sternum.

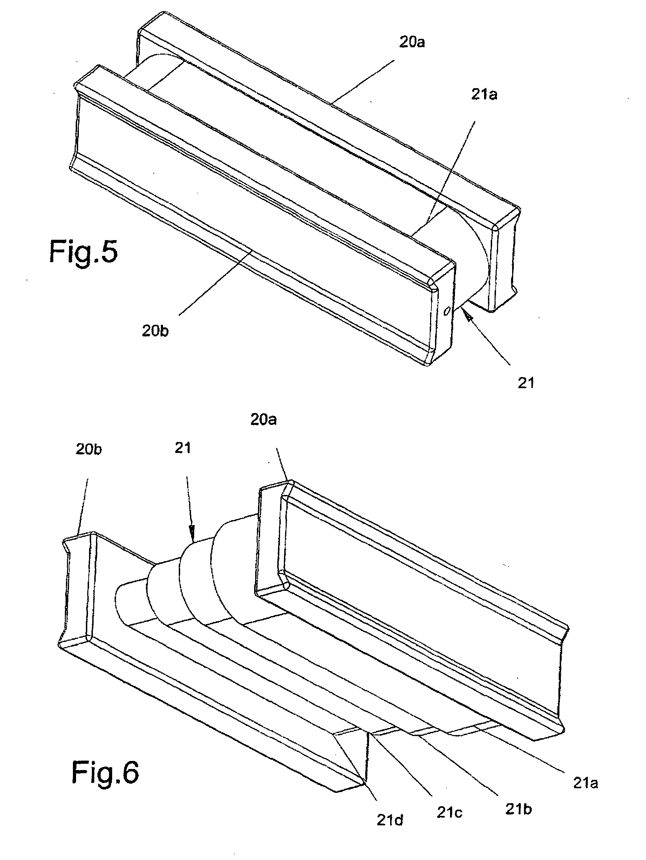

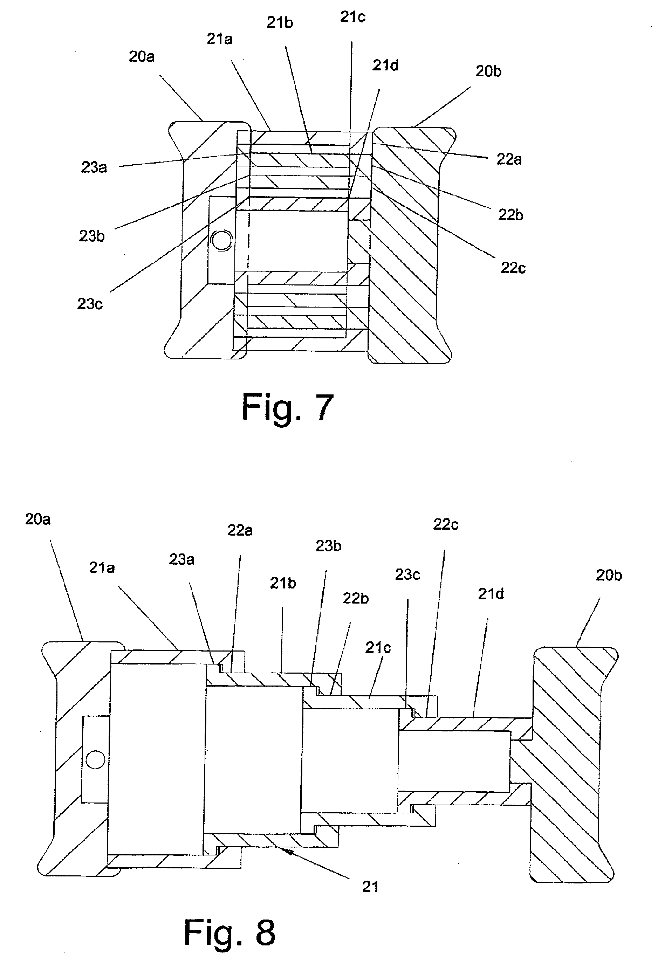

In a second embodiment not according to the invention, illustrated in figures 5-8, the two jaws 20a, 20b are connected on opposite sides to a telescopic actuator device, generically indicated by the numeral 21. The telescopic actuator device 21 is formed by a plurality (four in the embodiment shown, identified as 21a, b, c, d) of progressively smaller-sized sleeves such that they can be contained one inside the other. As illustrated more clearly in the cross-sections in figures 7 and 8, where the retractor device according to the invention is shown both in its closed condition (figure 7) and in its opened condition (figure 8), the widest terminal sleeve 21 a is rigidly connected to one of the two jaws (e.g. the jaw 20a), while the opposite, narrowest terminal sleeve (21d) is rigidly connected to the other jaw (e.g. the jaw 20b). The sleeves 21 a, b, c, d are slidingly connected to one another with a watertight connection, since the wider sleeves contain an internal flange (22a, b, c) that slides to form a watertight seal against the outer surface of the narrower sleeves, which in turn have an external flange 23a, b, c that slides to form a watertight seal against the inner surface of the wider sleeves. Here again, the mutual sliding between the sleeves 21 a,d is achieved by feeding/withdrawing an actuator fluid, such as a biocompatible liquid, in/from the internal chamber delimited by the sleeves 21 a,d through inlet/outlet ports.

The insertion of actuator fluid in the sleeves 21 a,d causes the divarication of the jaws 20a,b and the consequent moving away of the sternal stumps from one another, while the withdrawal of actuator fluid from said chamber enables the two jaws 20a,b to move closer together, due to the effect of the force exerted by the rib cage. Here again, the actuator fluid can be inserted manually, e.g. by means of a syringe, up until a maximum divarication has been reached corresponding to the maximum extension of each sleeve from the wider sleeve containing it, and then releasing the volume of fluid needed to achieve the degree of divarication required case by case.

It will be clear to a person skilled in the art that, in the two above-described embodiments of the invention, as an alternative to said manual methods, there may be automatic systems for feeding and withdrawing the actuator fluid, such as a metering pump on a feed line and a solenoid valve on a discharge line, the operation of which is governed by a microcontroller.

It should be noted that, in both the embodiments, the actuator has a very flat oval cross-section in order to restrict its overall dimensions in the direction orthogonal to the sternal plane.

Since the retractor device according to the invention is designed to be placed up against the heart, it is made using entirely biocompatible materials and all its components have rounded and/or bevelled edges to avoid any injury to the cardiac muscle even in the event of accidental contact.

The two jaws 1 a,b are shaped so as to guarantee an effective juxtaposition with the profile of the sternum and are wide enough to contain the surface pressure exerted thereon. Small vertical holes are created in the supporting surfaces to make easier the fixing of the retractor device to the sternal stumps and thus avoid any displacement of the retractor that might potentially be caused by movements of the rib cage and/or of the patient.

The device will be made in different sizes to guarantee that its overall longitudinal dimensions do not exceed the length of the sternum, and its dimensions in the direction orthogonal to the sternal plane do not extend too far beyond the edge of the surgical site in order to facilitate as far as possible the application of the sterile cover. Retractor devices may consequently be designed for use in neonatal surgery, for children and for various adult body sizes.

The actuator fluid is fed and withdrawn at the bottom of the sleeve 3 comprising the actuator, remaining fixed in position so that the fluid delivery tube emerges longitudinally with respect to the sterile cover on the patient's abdomen.

The retractor device according to the present invention enables numerous advantages to be achieved by comparison with the currently-adopted solutions. In the first place, it enables a safe and efficient sternum divarication to be achieved with a relatively standardisable procedure. Moreover, it enables the divarication amplitude to be adjusted from the outside without any need to reopen the surgical site, thus avoiding the removal of the protective membrane at any possible need. Depending on the patient's specific clinical needs, the operator can therefore increase or reduce the distance between the sternal stumps simply by taking action on the external syringe (or other actuator fluid insertion/withdrawal means) without removing the membrane. This versatility consequently also enables action to be taken promptly to deal with any deterioration in the patient's hemodynamic situation; on the other hand, it also enables a patient to be gradually "weaned" off the expanded sternal closure.

The retractor device according to the invention is made so as to guarantee absolutely sterile conditions and preferably of non-colonisable material. It may also be made in a resterilisable version or in a disposable version.

As concerns the primary use of the device according to the present invention for temporarily divaricating the sternum, it will be clear to a person skilled in the art that the device has other potential uses in the field of plastic surgery, in undermined lesions where a loss of substance makes it necessary to maintain the surface margins of the lesion open to enable in-depth wound irrigation from the outside, while reducing the distance between the margins as the tissue progressively grows back. The system is likewise applicable in all those modern surgery settings in which soft tissues need to be divaricated to enable the in-depth irrigation of a wound.

## Claims

1. A sternum retractor device suitable for temporarily maintaining a gap between the two sternal stumps in cardiosurgical procedures, comprising a pair of jaws (1a, 1b) configured in such a way to be inserted between the two sternal stumps and shaped so as to engage firmly on said stumps, actuating means (2) connected to said jaws for controlling their mutual closing and opening movements and means (16, 17) for the controlled feed or withdrawal of a biocompatible actuator fluid in/from said actuating means (2); and **characterized in that** said actuating means (2) take effect in a direction substantially perpendicular to the direction of the divarication of said stumps and are connected to said jaws (1a, 1b) by means of an articulated mechanism (7) that moves said jaws closer together or further away from one another as a result of said action, thus enabling the achievement and maintenance of the gap required between the two sternal stumps and also the progressive reduction of said gap as a function of the patient's condition.

2. A device according to claim 1, wherein said actuating means (2) comprise a fixed part (3) and a movable part (4) engaged with said fixed part, said articulated mechanism (7) comprising a first (10a, 10b) and a second pair (12a, 12b) of arms pivotally connected respectively to said fixed part (3) and said movable part (4), the arms of said first and second pairs being revolvingly connected, two by two, to the two jaws (1 a, 1 b), each pair on a respective common pin (9a, 9b; 11 a, 11 b).

3. A device according to claim 2, wherein the arms (10a, 10b) of said first pair extend from the end of a bar (8) attached crosswise to the end of said fixed part (3) opposite the end from which said movable part (4) extends.

4. A device according to claim 3, wherein the arms (12a, 12b) of said second pair are hinged onto a bracket (15) extending along said movable part (4) and at a distance therefrom that is greater than the thickness of a wall of said fixed part (3).

5. A device according to claims from 2 to 4, wherein said fixed part (3) is a tubular sleeve that is closed at one end and said movable part (4) is a piston with a flat oval cross-section slidingly contained inside the sleeve with a watertight seal.

6. A device according to any one of the claims from 1 to 5, wherein ports (5, 6) for the insertion/withdrawal of said actuator fluid are provided at the end of said fixed part (3) opposite the end from which said movable part (4) emerges.

7. A device according to any one of the claims 1 to 6, wherein said means for inserting/withdrawing said actuator fluid in or from said actuating means comprise a syringe (17) for its insertion and a shut-off valve (16) for its withdrawal, both operated manually.

8. A device according to any one of the claims from 1 to 6, wherein said means for inserting/withdrawing said actuator fluid in or from said actuating means comprise a dosing pump (17) and a solenoid valve (16) governed by a microcontroller.

9. A device according to any one of the previous claims, wherein said jaws (1a, 1 b) have a substantially U-shaped cross-section.

10. A device according to any one of the previous claims, wherein said jaws, said actuating means and said articulated mechanism have rounded or bevelled edges.

11. A device according to any of the claims 1 to 10, wherein said actuator fluid is a biocompatible liquid.

## Patentansprüche

1. Sternumretraktorvorrichtung, geeignet zum temporären Aufrechterhalten einer Lücke zwischen den zwei sternalen Stümpfen in herzchirurgischen Verfahren, enthaltend ein Paar von Backen (1a, 1b), die in einer solchen Weise konfiguriert sind, um zwischen die zwei sternalen Stümpfe eingefügt zu werden, und geformt sind, um fest an den zwei Stümpfen in Eingriff zu sein, Betätigungseinrichtungen (2), die an die Backen angeschlossen sind, um ihre gegenseitigen Schließ- und Öffnungsbewegungen zu steuern, und Einrichtungen (16, 17) für die gesteuerte Zuführung oder Abführung eines biokompatiblen Betätigungsfluides in die / von den Betätigungseinrichtungen (2), **dadurch gekennzeichnet, dass** die Betätigungseinrichtungen (2) in eine Richtung im Wesentlichen senkrecht zur Richtung des Auseinandergehens der Stümpfe wirken und mit den Backen (1a, 1b) mittels eines artikulierten Mechanismus (7) verbunden sind, der die Backen näher zusammen oder weiter voneinander weg bewegt als ein Ergebnis der Betätigung, womit das Erhalten und das Aufrechterhalten der Lücke, die zwischen den zwei sternalen Stümpfen benötigt wird, und auch die progressive Reduzierung der Lücke als eine Funktion des Zustandes des Patienten ermöglicht wird.

2. Vorrichtung nach Anspruch 2, wobei die Betätigungseinrichtungen (2) ein festes Teil (3) und ein bewegliches Teil (4) enthalten, das mit dem festen Teil in Eingriff ist, wobei der artikulierte Mechanismus (7) ein erstes (10a, 10b) und ein zweites Paar (12a, 12b) von Armen enthält, die schwenkbar jeweils mit dem festen Teil (3) und dem beweglichen Teil (4) verbunden sind, wobei die Arme des ersten und zweiten Paares drehbar paarweise mit den zwei Backen (1a, 1b) verbunden sind, jedes Paar an einem jeweiligen gemeinsamen Stift (9a, 9b; 11a, 11b).

3. Vorrichtung nach Anspruch 2, wobei sich die Arme (10a, 10b) des ersten Paares von dem Ende eines Stabes (8) erstrecken, der kreuzartig an dem Ende des festen Teils (3) entgegengesetzt zu dem Ende angebracht ist, von dem aus sich das bewegliche Teil (4) erstreckt.

4. Vorrichtung nach Anspruch 3, wobei die Arme (12a, 12b) des zweiten Paares an einer Halterung (15), die sich entlang des beweglichen Teils (4) erstreckt, und in einem Abstand angelenkt sind, der größer als die Dicke der Wand des festen Teils (3) ist.

5. Vorrichtung nach den Ansprüchen 2 bis 4, wobei das feste Teil (3) eine rohrartige Hülse ist, die an einem Ende geschlossen ist, und das bewegliche Teil (4) ein verschiebbar im Inneren der Hülse mit einer wasserdichten Abdichtung enthaltener Kolben mit einem flachen ovalen Querschnitt ist.

6. Vorrichtung nach einem der Ansprüche 1 bis 5, wobei Ports (5, 6) für das Zuführen / Abführen des Betätigungsfluides an dem Ende des festen Teils (3) entgegengesetzt dem Ende vorgesehen sind, von dem das bewegliche Teil (4) ausgeht.

7. Vorrichtung nach einem der Ansprüche 1 bis 6, wobei die Einrichtungen zum Zuführen / Abführen des Betätigungsfluides in die oder von den Betätigungseinrichtungen (2) eine Spritze (17) für seine Zuführung und ein Verschlussventil (16) für seine Abführung enthalten, die beide manuell betätigt werden.

8. Vorrichtung nach einem der Ansprüche 1 bis 6, wobei die Einrichtungen zum Zuführen / Abführen des Betätigungsfluides in die oder von den Betätigungseinrichtungen (2) eine Dosierpumpe (17) und ein Magnetventil (16) für seine Abführung enthalten, die von einem Mikrocontroller gesteuert werden.

9. Vorrichtung nach einem der vorhergehenden Ansprüche, wobei die Backen (1a, 1b; 20a, 20b) einen im Wesentlichen U-förmigen Querschnitt haben.

10. Vorrichtung nach einem der vorhergehenden Ansprüche, wobei die Backen, die Betätigungsmittel und der artikulierte Mechanismus abgerundete oder abgeschrägte Kanten haben.

11. Vorrichtung nach einem der Ansprüche 1 bis 10, wobei das Betätigungsfluid eine biokompatible Flüssigkeit ist.

## Revendications

1. Un dispositif de rétractation de sternum apte à maintenir temporairement un espace entre les deux tronçons sternaux dans des interventions cardio-chirurgicales, comprenant une paire des mâchoires (1a, 1 b) configurées de façon à être aptes à être insérées entre les deux tronçons sternaux et conformées de façon à coopérer fermement avec lesdits tronçons, des moyens de commande (2) reliés aux dites mâchoires pour commander leur mouvement mutuel de fermeture et d'ouverture et des moyens (16, 17) pour contrôler l'alimentation en un fluide actionneur biocompatible des dits moyens commande (2) ou le retrait du fluide actionneur à partir de ceux-ci ; et **caractérisé en ce que** lesdits moyens de commande (2) agissent dans une direction sensiblement perpendiculaire à la direction d'écartement des dits tronçons et sont reliés aux dites mâchoires (1a, 1b) au moyen d'un mécanisme articulé (7) qui déplace lesdites mâchoires en les rapprochant ou en les éloignant l'une de l'autre en fonction de ladite action, permettant de ce fait la réalisation et le maintien de l'espace exigé entre les deux tronçons sternaux ainsi que la réduction progressive du dit espace en fonction de l'état du patient.

2. Un dispositif selon la revendication 1, où lesdits moyens de commande (2) comportent une partie fixe (3) et une partie mobile (4) coopérant avec ladite partie fixe, ledit mécanisme articulé (7) comprenant une première paire (10a, 10b) et une seconde paire (12a, 12b) de bras montées pivotantes respectivement sur ladite partie fixe (3) et sur ladite partie mobile (4), les bras des dites première et seconde paires étant reliés de façon pivotante, deux par deux, aux deux mâchoires (1a, 1b), chaque paire sur une goupille respective commune (9a, 9b ; 11 a, 11 b).

3. Un dispositif selon la revendication 2, où les bras (10a, 10b) de ladite première paire s'étendent à partir de l'extrémité d'une barre (8) fixée de façon transversale à l'extrémité de ladite partie fixe (3) opposée à l'extrémité à partir de laquelle s'étend ladite partie mobile (4).

4. Un dispositif selon la revendication 3, où les bras (12a, 12b) de ladite seconde paire sont articulés sur une équerre (15) s'étendant le long de ladite partie mobile (4) et à une distance de celle-ci qui est plus grande que l'épaisseur d'une paroi de ladite partie fixe (3).

5. Un dispositif selon l'une des revendications 2 à 4, où ladite partie fixe (3) est un manchon tubulaire qui est fermé à une extrémité et où ladite partie mobile (4) est un piston d'une section transversale ovale plate monté de façon coulissante à l'intérieur du manchon avec un joint étanche.

6. Un dispositif selon l'une quelconque des revendications 1 à 5, où des ports (5, 6) pour alimenter en dit fluide actionneur ou le retirer sont prévus à l'extrémité de ladite partie fixe (3) opposée à l'extrémité à partir de laquelle émerge ladite partie mobile (4).

7. Un dispositif selon l'une quelconque des revendications 1 à 6, où lesdits moyens pour alimenter en fluide actionneur lesdits moyens de commande ou le retirer de ceux-ci comportent une seringue (17) pour l'alimentation et une valve d'arrêt (16) pour le retrait, toutes deux étant actionnées manuellement.

8. Un dispositif selon l'une quelconque des revendications 1 à 6, où lesdits moyens pour alimenter en fluide actionneur lesdits moyens de commande ou le retirer de ceux-ci comportent une pompe de dosage (17) et une vanne électromagnétique (16) commandées par un microcontrôleur.

9. Un dispositif selon l'une quelconque des revendications précédentes, où lesdites mâchoires (1a, 1b) ont une section transversale sensiblement en forme de U.

10. Un dispositif selon l'une quelconque des revendications précédentes, où lesdites mâchoires, lesdits moyens de commande et ledit mécanisme articulé ont des bords arrondis ou biseautés.

11. Un dispositif selon l'une quelconque des revendications 1 à 10, où ledit fluide actionneur est un liquide biocompatible.
